# FASCICULE DE BREVET EUROPEEN

(11) **EP 0 887 073 B1**
(45) Date de publication et mention de la délivrance du brevet: **08.01.2003**
(21) Numéro de dépôt: 98401574.3
(22) Date de dépôt: 25.06.1998
(51) Int. Cl.: A61K 7/48, A61K 7/06, A61K 7/027, A61K 7/043

(54) **Composition cosmétique ou dermatologique contenant au moins une silicone fluorée à chaîne alkyle**
Kosmetische oder dermatologische Zusammensetzung die mindestens ein Fluorosilikon mit Alkylkette enthält
Cosmetic or dermatological composition containing at least an alkyl chain bearing fluorosilicone

(30) Priorité: 26.06.1997 FR 9708027
(43) Date de publication de la demande: 30.12.1998
(73) Titulaire: L'OREAL, 75008 Paris (FR)
(72) Inventeur: Arnaud, Pascal, 94240 L'Hay les Roses (FR); Bara, Isabelle, 75013 Paris (FR)
(74) Mandataire: Tanty, François

(56) Documents cités:
- EP-A- 0 511 092
- EP-A- 0 640 644
- EP-A- 0 657 486
- US-A- 5 473 038

## Description

La présente invention a pour objet une composition, cosmétique ou dermatologique, contenant une silicone fluorée à chaîne alkyle en tant qu'ingrédient capable d'en améliorer diverses propriétés essentielles.

On a déjà proposé l'utilisation de composés fluorés dans des compositions cosmétiques pour leurs propriétés filmogènes, ainsi que pour leurs propriétés de douceur et de rémanence à l'eau.

Ainsi, il a été décrit dans EP-390206 et EP-494412, l'utilisation de polymères hydrocarbonés perfluorés dans divers types de compositions cosmétiques. Toutefois, ces polymères hydrocarbonés perfluorés présentent un spectre de compatibilité chimique, notamment avec les corps gras, relativement restreint, limitant ainsi le choix des matières premières avec lesquelles ils peuvent être associés, et la nature des compositions les contenant. De plus, ils ne peuvent être présents qu'en proportion relativement faible dans les compositions cosmétiques.

Il a également été proposé dans JP-7-103582 et JP 2-295913, l'utilisation de silicones fluorées dans des compositions cosmétiques mais celles-ci présentent également des spectres de compatibilité relativement restreints, limitant de même le choix des ingrédients avec lesquels ils peuvent être associés, ainsi que les proportions dans lesquelles ils peuvent être présents dans les compositions.

On a maintenant découvert qu'une famille particulière de silicones fluorées, à savoir des silicones fluorées, à chaîne alkyle en C₆-C₂₂ (ci-après désignées par alkylsilicones fluorées), présentait des propriétés intéressantes en vue d'un usage cosmétique ou dermatologique. Les alkylsilicones fluorées peuvent être notamment de bons agents filmogènes conduisant, après application, à la formation d'un film à la fois homogène, continu, rémanent à l'eau, et présentant en outre un excellent compromis entre sa durabilité et sa facilité d'élimination. Les alkylsilicones fluorées des compositions selon l'invention constituent également des agents de lubrification ainsi que des agents liants très satisfaisants.

De plus, ces alkylsilicones fluorées présentent un spectre de compatibilité beaucoup plus étendu que ceux des composés fluorés tels que les silicones fluorées et les perfluoroéthers décrits dans l'art antérieur, ceci permettant de réaliser des compositions à l'aide de substances qui ne pouvaient être utilisées jusqu'à présent.

Elles peuvent en outre être introduites dans les compositions en une proportion plus importante sans pour autant en affecter leur homogénéité et leurs autres propriétés mais tout au contraire les améliorer.

La présente invention a donc pour objet une composition cosmétique ou dermatologique contenant en tant qu'ingrédient au moins une alkylsilicone fluorée répondant à l'une des formules (I) et/ou (II) suivantes : 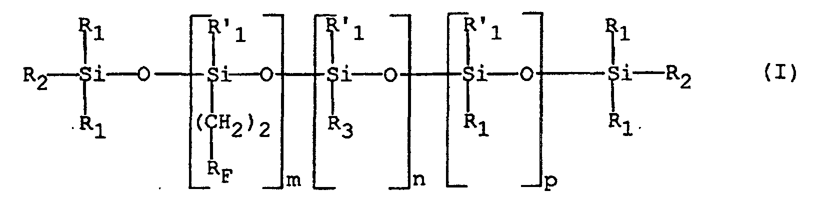 dans laquelle :
R₁ et R'₁ représentent indépendamment un radical alkyle, linéaire ou ramifié, ayant de 1 à 6 atomes de carbone ou un radical phényle,
R₂ représente R₁, -OH, ou -(CH₂)_{f}-R_{F}, f étant un nombre entier allant de 0 à 10,
R₃ représente un radical alkyle, linéaire ou ramifié, ayant de 6 à 22 atomes de carbone,
R_{F} représente un radical de formule -(CF₂)_{q}-CF₃, q étant un nombre entier allant de 0 à 10,
m et n représentent un nombre entier allant de 1 à 50, et
p représente un nombre entier allant de 0 à 2000, 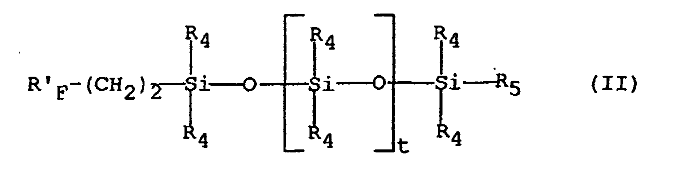
dans laquelle :
R₄ représente un radical alkyle, linéaire ou ramifié, ayant de 1 à 6 atomes de carbone, ou un radical phényle,
R₅ représente un radical alkyle, linéaire ou ramifié, ayant de 6 à 22 atomes de carbone,
R'_{F} représente un radical de formule -(CF₂)ₛ-CF₃, s étant un nombre entier allant de 0 à 15, et
t représente un nombre entier allant de 1 à 2000.

Selon un mode de réalisation particulier des compositions cosmétiques selon l'invention l'alkylsilicone fluorée répond à la formule (I) dans laquelle :
R₁, R'₁ et R₂ représentent le radical méthyle,
R₃ représente un radical alkyle linéaire ayant de 6 à 22 atomes de carbone,
m et n sont des nombres entiers allant de 1 à 20, et
q est un nombre entier allant de 1 à 13.

Selon un autre mode de réalisation des compositions selon l'invention l'alkylsilicone fluorée répond à la formule (II) dans laquelle :
R₄ représente le radical méthyle,
R₅ représente un radical alkyle linéaire, ayant de 6 à 22 atomes de carbone, et
s représente un nombre entier allant de 1 à 13.

Les alkylsilicones fluorées telles que définies ci-dessus sont des composés connus qui ont été décrits notamment dans le brevet US-5,473,038.

Dans les compositions selon l'invention, l'alkylsilicone fluorée telle que définie ci-dessus est généralement présente en une proportion allant de 0,1 à 99 % en poids, mais de préférence de 1 à 80 % en poids par rapport au poids total de la composition.

Selon un premier mode de réalisation particulier des compositions selon l'invention, celles-ci sont anhydres et comprennent une phase grasse en une proportion allant de 0,1 à 99,9 % en poids par rapport au poids total de la composition, ladite phase grasse contenant :
- (i) de 0,1 à 99,9 % en poids, par rapport au poids total de ladite composition, d'une alkylsilicone fluorée de formule (I) et/ou (II), et
- (ii) de 0 à 99,8 % en poids par rapport au poids total de ladite composition, d'au moins un corps gras liquide, solide ou semi-solide.

Ce ou ces corps gras peuvent être choisis parmi les huiles, les cires, les gommes et/ou les corps gras dits pâteux.

### A- Les huiles de la phase grasse peuvent être d'origine minérale, animale, végétale ou de synthèse, celles-ci pouvant être volatiles ou non à température ambiante.

Comme huile d'origine minérale, on peut citer notamment l'huile de paraffine et l'huile de vaseline.

Comme huile d'origine animale, on peut citer notamment le squalane ou perhydrosqualène.

Comme huile d'origine végétale, on peut citer notamment l'huile d'amande douce, l'huile de calophyllum, l'huile de palme, l'huile d'avocat, l'huile de jojoba, l'huile de sésame, l'huile d'olive, l'huile de ricin et les huiles de germes de céréales telles que par exemple l'huile de germes de blé.

Comme huile de synthèse, on peut citer notamment :
(1) les esters de formule :

   R₆-COOR₇

   dans laquelle :
   R₆ représente le reste d'un acide gras supérieur ayant de 7 à 20 atomes de carbone et
   R₇ représente un radical hydrocarboné ayant de 3 à 30 atomes de carbone.

   Parmi ces esters, on peut notamment citer : l'huile de Purcellin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, l'isononanoate d'isononyle, les esters dérivés de l'acide lanolique tels que le lanolate d'isopropyle et le lanolate d'isocétyle.
   Comme autres huiles de synthèse, on peut citer en outre l'isododécane, l'isohexadécane, les polyisobutènes et le polyisobutène hydrogéné ainsi que les acétylglycérides, les octanoates et décanoates de polyalcools tels que ceux de glycol et de glycérol, les ricinoléates d'alcools et de polyalcools tels que celui d'alcool cétylique, le dicaprylate de propylène glycol et l'adipate de diisopropyle ;
(2) les alcools gras tels que l'alcool oléique, l'alcool linoléique, l'alcool linolénique, l'alcool isostéarylique et l'octyldodécanol ;
(3) les huiles de silicone telles que les polydiorganosiloxanes linéaires éventuellement fonctionnalisés, les polydiorganosiloxanes cycliques et en particulier les cyclotétra- et penta-diméthicones et les organopolysiloxanes tels que des alkyl, alcoxy ou phényl diméthicones et en particulier la phényltriméthicone ;
(4) les huiles fluorées telles que les perfluoroalcanes et les perfluoropolyéthers et les huiles hydrocarbonées partiellement fluorées.

### B- Les cires de la phase grasse peuvent être d'origine minérale, fossile, animale, végétale, de synthèse ou bien encore être des huiles hydrogénées ou des esters gras concrets à 25°C.

Parmi les cires minérales, on peut citer notamment les cires microcristallines, la paraffine, la vaseline et la cérésine.

Parmi les cires fossiles, on peut citer l'ozokérite et la cire de montan.

Parmi les cires d'origine animale, on peut citer la cire d'abeilles, le spermaceti, la cire de lanoline ainsi que les dérivés issus de la lanoline tels que les alcools de lanoline, la lanoline hydrogénée, la lanoline hydroxylée, la lanoline acétylée, les acides gras de lanoline et l'alcool de lanoline acétylé.

Parmi les cires d'origine végétale, on peut citer notamment la cire de candellila, la cire de carnauba, la cire du Japon et le beurre de cacao.

Parmi les cires de synthèse, on peut citer notamment les homopolymères d'éthylène et les copolymères d'éthylène et d'un monomère répondant à la formule

CH₂=CH-R₈ (III)

dans laquelle :
R₈ représente un radical alkyle ayant de 1 à 30 atomes de carbone, un radical aryle ou aralkyle.

Le radical alkyle ayant de 1 à 30 atomes de carbone est de préférence le radical méthyle, éthyle, propyle, isopropyle, butyle, décyle, dodécyle ou octadécyle.

On peut également utiliser les cires obtenues par synthèse Fisher-Tropsch ainsi que les cires de silicone.

Parmi les huiles hydrogénées concrètes à 25°C, on peut citer notamment l'huile de ricin hydrogénée, l'huile de palme hydrogénée, le suif hydrogéné et l'huile de coco hydrogénée.

Parmi les esters gras concrets à 25°C, on peut citer notamment le mono-myristate de propylène glycol et le myristate de myristyle.

Comme cires utilisables dans les compositions selon l'invention, on peut encore citer l'alcool cétylique, l'alcool stéarylique, les mono-, di- et triglycérides concrets à 25°C, le monoéthanolamide stéarique, la colophane et ses dérivés tels que les abiétates de glycol et de glycérol, les sucro-glycérides et les oléates, myristates, lanolates, stéarates et dihydroxystéarates de calcium, de magnésium, de zinc et d'aluminium.

### C- Les corps gras de type pâteux peuvent être d'origine minérale, animale, végétale ou de synthèse.

Parmi les corps gras pâteux, on peut citer notamment les esters de synthèse tels que le propionate d'arachidyle, le polylaurate de vinyle, les cires de polyéthylène et les organopolysiloxanes tels que les alkyldiméthicones, les alcoxydiméthicones ou les esters diméthicones.

Des différents corps gras énumérés ci-dessus, on utilise de préférence selon l'invention les suivants :
(1) Parmi les huiles : l'isododécane, le polyisobutène hydrogéné, le squalane, l'isononanoate d'isononyle, les cyclométhicones telles que les cyclotétra- et pentadiméthicones et la phényltriméthicone ;
(2) Parmi les cires :
   - (i) les homopolymères d'éthylène ayant une masse moléculaire moyenne en poids comprise entre 200 et 1000 et notamment ceux commercialisés sous les dénominations de "Polywax 500" et "Polywax 655" par la Société BARECO,
   - (ii) les copolymères d'éthylène et d'au moins un monomère de formule (III) dans laquelle le radical R₈ représente un radical méthyle, éthyle, isopropyle, butyle, dodécyle ou octadécyle, ces copolymères ayant une masse moléculaire moyenne en poids comprise entre 200 et 1.000. Parmi ceux-ci, on peut citer les copolymères éthylène-propylène tels que ceux commercialisés sous les dénominations de "Petrolite CP-7" et "Petrolite CP-12" par la Société BARECO, et les copolymères éthylène-hexène tels que ceux commercialisés sous les dénominations de "Petrolite CH-7" et "Petrolite CH-12" par la Société BARECO,
   - (iii) les cires microcristallines et l'ozokérite,
   - (iv) la cire d'abeilles ;
(3) Comme corps gras pâteux, le propionate d'arachidyle.

Les compositions anhydres selon l'invention peuvent bien entendu contenir en outre un ou plusieurs additifs ou adjuvants cosmétiques ou dermatologiques conventionnels.

Ces compositions anhydres peuvent se présenter sous différentes formes telles que notamment sous la forme d'un gel huileux, de produits solides tels que des poudres compactées ou coulées, ou bien encore de sticks tels que par exemple des rouges à lèvres.

Lorsque les compositions selon l'invention se présentent sous forme d'un gel huileux, elles contiennent généralement, outre les constituants définis précédemment, un gélifiant huileux.

Parmi les gélifiants huileux, on peut notamment citer les esters métalliques tels que le stéarate de polyoxyaluminium et l'hydroxystéarate d'aluminium ou de magnésium, les esters d'acide gras et de glycol, les triglycérides, les mélanges d'alcools gras, les dérivés de cholestérol et en particulier l'hydroxycholestérol, et les minéraux argileux gonflants en présence d'huile et en particulier ceux appartenant au groupe des montmorillonites.

Les gélifiants huileux peuvent être présents en une proportion très variable selon la texture des compositions recherchée. Toutefois, dans la plupart des cas, ils sont présents en une proportion allant de 0,1 à 30 % en poids par rapport au poids total de la composition.

Ces compositions anhydres selon l'invention peuvent être utilisées notamment comme produits de soins, de nettoyage, de démaquillage ou de maquillage.

Lorsqu'elles se présentent sous forme de produits de maquillage, elles peuvent être en particulier des fonds de teint, des mascaras, des eyeliners, des rouges à lèvres, des fards à paupières ou à joues. Ces compositions sont généralement colorées et contiennent alors en tant qu'adjuvants cosmétiques, des colorants et/ou des pigments bien connus dans le domaine des produits de maquillage.

Selon un deuxième mode de réalisation des compositions selon l'invention, celles-ci sont des dispersions sous forme d'une émulsion eau-dans-l'huile (E/H) ou huile-dans-l'eau (H/E), stable, qui sont essentiellement constituées (i) d'une phase grasse en une proportion allant de 0,1 à 50 % en poids par rapport au poids total de la composition, ladite phase grasse contenant une alkylsilicone fluorée de formule (I) et/ou (II) telle que définie ci-dessus en une proportion allant de 0,1 à 50 % en poids par rapport au poids total de la composition, (ii) d'une phase aqueuse en une proportion allant de 50 à 98,9 % en poids par rapport au poids total de la composition, et (iii) d'au moins un agent émulsionnant en une proportion allant de 1 à 10 % en poids par rapport au poids total de la composition sous forme d'émulsion.

On a constaté que contrairement aux huiles fluorées utilisées antérieurement, les alkylsilicone fluorées de formule (I) et/ou (II) permettaient d'obtenir des émulsions de très bonne stabilité dans le temps et ceci quelles que soient les conditions de stockage.

Comme agent émulsionnant ou tensioactif pouvant être utilisé dans les compositions selon l'invention du type émulsion E/H ou H/E, on peut citer notamment les agents tensioactifs siliconés, et en particulier ceux appartenant à la famille des alkyl- ou alcoxydiméthicone copolyols. Parmi les alkyl- ou alcoxydiméthicone copolyols, on peut citer notamment les composés répondant à la formule générale suivante : 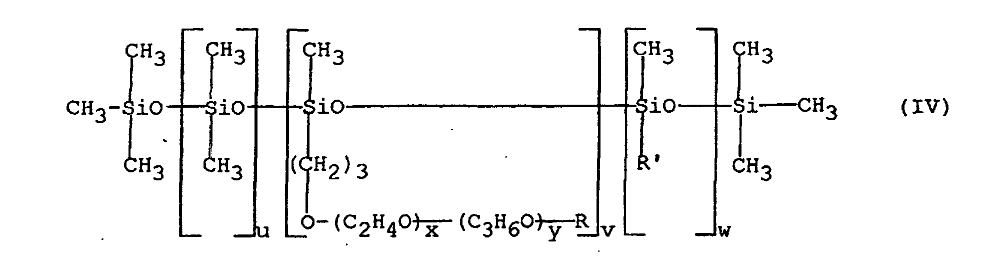 dans laquelle :
R est un atome d'hydrogène, un alkyle en C₁-C₁₆, un alcoxy ou acyle,
R' est un radical alkyle ou alcoxy en C₈-C₂₂,
u = 0 à 200,
v = 1 à 40,
w = 1 à 100,
le poids moléculaire du radical 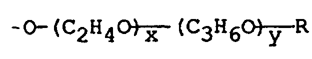 étant de 250 à 2000, x et y étant choisis de telle sorte que le rapport en poids des groupes oxyéthylène/oxypropylène soit compris entre 100:0 et 20:80.

Parmi les produits du commerce pouvant contenir tout ou partie des alkyldiméthicones copolyols utilisables selon l'invention, on peut citer notamment ceux vendus sous les dénominations de "Abil WE09", "Abil EM90" ou "Abil WS08" par la Société GOLDSCHMIDT, de "Q2 5200" ou "Q2 3225C" par la Société DOW CORNING et de "218 1138" par la Société GENERAL ELECTRIC.

Ces émulsions se présentent de préférence sous forme de crèmes et peuvent être utilisées comme produits de soin, de nettoyage ou de maquillage.

Elles peuvent en outre constituer des produits de protection de la peau contre divers types d'agression grâce à l'excellente qualité du film formé par l'alkylsilicone fluorée de formule (I) et/ou (II) après application. Les compositions selon l'invention peuvent également constituer d'excellents produits solaires lorsqu'on y introduit des filtres solaires UVA et/ou UVB et/ou des pigments de taille nanométrique.

Lorsque ces compositions sont des produits de maquillage, celles-ci peuvent être notamment des fonds de teint contenant alors en une certaine proportion des pigments et/ou des colorants.

Les compositions selon l'invention, qu'elles soient du type anhydre ou sous forme de dispersion, présentent d'excellentes propriétés cosmétiques telles que notamment une meilleure facilité d'application, une grande douceur et conduisent à l'obtention d'un maquillage homogène.

Ces compositions de maquillage présentent par ailleurs une excellente résistance au transfert vers un support autre que celui sur lequel elles ont été appliquées. Par transfert, on entend le déplacement d'une fraction de la composition par contact avec un autre support, qu'il soit de même nature ou de nature différente. Cette propriété est bien entendu particulièrement intéressante lorsque les compositions sont fortement colorées. Par exemple, lorsque les compositions de maquillage sont des fards à paupières, des eyeliners ou des mascaras, cette propriété évite un transfert des compositions sur les mains par frottement ou par contact de celles-ci avec les yeux. Pour les compositions sous forme de rouges à lèvres, cette propriété permet de limiter le transfert du rouge à lèvres sur les mains ou encore sur les joues d'une autre personne. Par ailleurs, cette propriété permet également d'éviter de tacher les serviettes de table, et de laisser des empreintes sur les verres, tasses et autres.

Lorsque les compositions selon l'invention sont des fonds de teint, cette propriété permet notamment de prévenir le transfert du fond de teint sur les cols des chemisiers et d'éviter ainsi de les tacher.

Selon un troisième mode de réalisation des compositions selon l'invention, celles-ci sont sous forme de produits pour les ongles tels que des vernis à ongles ou des produits pour les soins des ongles comprenant une alkylsilicone fluorée de formule (I) et/ou (II) telle que définie précédemment en une proportion allant de 0,1 à 99,9 % en poids par rapport au poids total de la composition.

De préférence, selon ce mode de réalisation, les compositions sont sous forme de vernis à ongles et contiennent :
- (i) une alkylsilicone fluorée de formule (I) et/ou (II) en une proportion allant de 2 à 40 % en poids par rapport au poids total du vernis,
- (ii) un système solvant pour vernis, et
- (iii) une substance filmogène.

Selon ce mode de réalisation, le système solvant du vernis est généralement présent en une proportion allant de 55 à 90 % en poids par rapport au poids total du vernis.

Bien que le système solvant puisse être du type aqueux, celui-ci est de préférence constitué par un mélange de divers solvants organiques volatiles, ceci en vue d'obtenir des temps de séchage relativement courts.

Parmi ces solvants, on peut citer l'acétone, l'acétate d'éthyle, l'acétate de butyle, l'acétate de méthoxy-2 éthyle, la méthyl-éthyl-cétone, la méthyl-isobutyl-cétone, l'acétate de méthyle, l'acétate d'amyle et l'acétate d'isopropyle.

Le système solvant peut comprendre également un diluant qui est de préférence un hydrocarbure, linéaire ou ramifié, saturé, tel que l'hexane ou l'octane, ou encore un hydrocarbure aromatique tel que le toluène ou le xylène dans une proportion allant généralement de 10 à 35 % en poids par rapport au poids total du vernis. Le système solvant peut également inclure d'autres solvants volatiles tels que l'éthanol, le n-butanol, le n-propanol, l'isopropanol ou leurs mélanges.

Outre l'alkylsilicone fluorée de formule (I) et/ou (II), la composition selon l'invention peut également comprendre une substance filmogène. Cette substance filmogène est généralement présente en une proportion allant de 5 à 35 % en poids par rapport au poids total du vernis. Parmi ces substances filmogènes, on peut citer les nitrocelluloses du type "RS" ou "SS" et en particulier la nitrocellulose type 1/4 seconde RS, la nitrocellulose type 1/2 seconde RS, la nitrocellulose type 1/2 seconde SS et la nitrocellulose type 3/4 seconde RS. Comme substance filmogène, on peut également utiliser selon l'invention des dérivés polyvinyliques tels que le butyrate de polyvinyle.

Comme autre substance filmogène, on peut utiliser selon l'invention des dérivés cellulosiques différents de la nitrocellulose, des polymères ou copolymères acryliques, des résines du type acrylique, styrénique, acrylate-styrénique et vinylique, des copolymères vinyliques, des polymères polyesters, des résines du type aryl-sulfonamide et des résines alkydes.

Les vernis selon l'invention peuvent également contenir un agent plastifiant qui est généralement présent en une proportion allant de 5 à 20 % en poids par rapport au poids total du vernis. Les agents plastifiants permettent de régler la flexibilité du film sans affaiblir sa résistance ou sa force physique. Parmi les agents plastifiants, on peut citer : le phosphate de tricrésyle, le benzoate de benzyle, le phosphate de tributyle, l'acétyl ricinoléate de butyle, le citrate de triéthyle, l'acétyl citrate de tributyle, le phtalate de dibutyle et le camphre.

Les produits pour les ongles selon l'invention peuvent être soit incolores soit colorés. Lorsqu'ils sont colorés, ils contiennent alors des pigments et/ou des colorants bien connus dans le domaine des vernis à ongles.

Selon un quatrième mode de réalisation particulier des compositions selon l'invention, celles-ci sont des compositions capillaires contenant dans un véhicule cosmétique choisi parmi les solutions alcooliques et hydroalcooliques, au moins une alkylailicone fluorée de formule (I) et/ou (II) telle que définie précédemment en une proportion allant de 0,5 à 40 % en poids par rapport au poids total de la composition.

L'alcool est de préférence l'éthanol ou l'isopropanol, généralement présent dans les solutions hydroalcooliques en une proportion allant de 60 à 99,5 % en poids par rapport au poids total.

Ces compositions capillaires sous forme de lotions à pulvériser ou encore d'aérosols s'appliquent facilement et forment un film continu et homogène, permettant un gainage très satisfaisant des cheveux, qui s'élimine néanmoins facilement au shampooing. Les compositions capillaires selon l'invention peuvent en outre contenir divers additifs et ingrédients utilisés dans le domaine du traitement des cheveux tels que notamment des filtres UV, et constituer ainsi des compositions destinées notamment à protéger les cheveux teints des effets néfastes du soleil.

Bien qu'il ait été fait référence ci-dessus plus particulièrement à des lotions, les compositions capillaires selon l'invention peuvent également se présenter sous forme d'émulsions fluides permettant une bonne imprégnation de la chevelure.

Les compositions selon l'invention, telles qu'elles viennent d'être décrites ci-dessus, peuvent contenir en outre un ou plusieurs adjuvants cosmétiques conventionnels tels que des vitamines, des hormones, des agents antioxydants, des conservateurs, des charges, des parfums, des épaississants, des agents hydratants, des agents humectants, des polymères anioniques, non-ioniques ou amphotères, ou des actifs cosmétiques ou dermatologiques.

Parmi ces adjuvants, les charges sont généralement présentes dans les produits de maquillage ou de soins ou dans les produits dermatologiques en une proportion maximum d'environ 99,9 % en poids par rapport au poids total de la composition.

Ces charges, sous forme de poudres très fines, peuvent être d'origine naturelle ou synthétique. Parmi celles-ci, on peut notamment citer :
a) les poudres minérales telles que le talc, le kaolin, le mica, la silice, les silicates, l'alumine, les zéolites, l'hydroxyapatite, la séricite, le dioxyde de titane, les micatitanes, le sulfate de baryum, l'oxychlorure de bismuth, le nitrure de bore et les poudres métalliques telles que la poudre d'aluminium ;
b) les poudres végétales telles que les poudres d'amidon de mais, de froment ou de riz ;
c) les poudres organiques telles que les poudres de nylon, de polyamide, de polyester, de polytétrafluoroéthylène ou de polyéthylène.

Ces différentes poudres peuvent en outre être enrobées par exemple par des sels métalliques d'acides gras, des acides aminés, de la lécithine, du collagène, des composés siliconés, des composés fluorés, ou par tout autre enrobage usuel.

Outre les charges, les colorants et pigments entrent également non seulement dans les compositions de maquillage anhydres ou sous forme de dispersions mais également dans les vernis à ongles. Les colorants et/ou pigments sont généralement présents en une proportion maximum d'environ 40 % par rapport au poids total de la composition.

Dans les rouges à lèvres, la proportion en au moins un colorant et/ou pigment est généralement d'environ 0,1 à 15 % en poids par rapport au poids total du rouge à lèvres.

Parmi les colorants des produits de maquillage et notamment des rouges à lèvres, on peut citer les dérivés d'éosine tels que le D&C Red n° 21 et les dérivés de fluorescéine halogénés tels que le D&C Red n°27, le D&C Red Orange n°5 en association avec le D&C Red n°21 et le D&C Orange n°10.

Parmi les pigments qui peuvent être minéraux ou organiques ou encore des laques métalliques, on peut citer le dioxyde de titane, l'oxyde de zinc, le D&C Red n°36 et le D&C Orange n°17, les laques de calcium de D&C Red n°7, 11, 31 et 34, la laque de baryum de D&C Red n°12, la laque de strontium D&C Red n°13, les laques d'aluminium de FD&C Yellow n°5, de FD&C Yellow n°6, de D&C Red n°27, de D&C Red n°21, de FD&C Blue n°1, les oxydes de fer, le violet de manganèse, l'oxyde de chrome et le bleu d'outremer.

Parmi les pigments des vernis à ongles les plus employés, on peut citer les D&C Red n° 8, 10, 30 et 36, les laques de baryum des D&C Red n°6, 9 et 12, les laques de calcium des D&C Red n°7, 11, 31 et 34, la laque de strontium du D&C Red n°30 ainsi que le D&C Orange n°17 et le D&C Blue n°6.

Les vernis à ongles peuvent également contenir du dioxyde de titane en vue d'impartir aux vernis une certaine opacité ainsi que certaines substances iridescentes telles que la guanine et des produits permettant d'éviter la sédimentation des pigments tels que des argiles de montmorillonite modifiées comme par exemple la Bentone 27, la Bentone 34 ou la Bentone 38.

L'invention sera maintenant illustrée par les différents exemples suivants dont les quantités sont exprimées en poids.

### EXEMPLE 1 : Rouge à lèvres résistant au transfert

On prépare un rouge à lèvres en procédant au mélange des ingrédients suivants :

### Phase A

- Alkylsilicone fluorée (a) 20 g
- Propionate d'arachidyle 9 g
- Homopolymère d'éthylène commercialisé sous la dénomination de "Polywax 500" par la Société BARECO 16 g

### Phase B

- Cyclotétradiméthicone commercialisée sous la dénomination de "Dow Corning 244 Fluid" par la Société DOW CORNING 46 g

### Phase C

- Pigments 9 g

### (a) Alkylsilicone fluorée de formule (I) dans laquelle R₁, R'₁ et R₂ représentent un radical méthyle, R₃ représente un radical alkyle linéaire ayant 16 atomes de carbone, R_{F} représente -(CF₂)₈ - CF₃, q est égal à 5, m, n et p sont respectivement égaux à 10, 20 et 50.

Ce rouge à lèvres est préparé par chauffage des ingrédients de la *Phase A* à une température d'environ 95°C. Après fusion complète, on ajoute les pigments *(Phase C)* puis, à 60°C, la *Phase B.* On homogénéise ensuite à l'aide d'une turbine de type Moritz à la vitesse de 3.000 t/mn. On peut alors couler le mélange homogène obtenu à 85°C dans des alvéoles pour rouges à lèvres.

Après refroidissement, les rouges à lèvres sont retirés des alvéoles.

Les rouges à lèvres obtenus s'appliquent très facilement sur les lèvres, auxquelles ils confèrent une très grande douceur. Les rouges à lèvres, après application présentent en outre une excellente tenue, c'est-à-dire une excellente résistance à l'usure. De plus, ils possèdent une bonne résistance au transfert.

### EXEMPLE 2 : Rouge à lèvres résistant au transfert

Ce rouge à lèvres est obtenu selon le même mode opératoire que celui décrit à l'exemple 1 à partir des phases suivantes :
Phase A
   - Alkylsilicone fluorée (b) 20 g
   - Propionate d'arachidyle 9 g
   - Homopolymère d'éthylène commercialisé sous la dénomination de "Polywax 500" par la Société BARECO 16 g
*Phase B*
   - Cyclotétradiméthicone commercialisée sous la dénomination de "DOW CORNING 244 fluid" par la Société DOW CORNING 46 g
*Phase C*
   - Pigments 9 g

### (b) Alkylsilicone fluorée de formule (II) dans laquelle R₄ représente un radical méthyle, R₅ représente un radical alkyle linéaire ayant 16 atomes de carbone, R'_{F} représente -(CF₂)₈-CF₃ et t est égal à 1.

Ce rouge à lèvres qui se différencie du précédent par la nature de l'alkylsilicone fluorée présente d'excellentes propriétés de tenue et de confort.

### EXEMPLE 3 : Rouge à lèvres résistant au transfert

Selon la méthode conventionnelle de préparation des rouges à lèvres on a procédé au mélange des différents ingrédients suivants :
- Alkylsilicone fluorée (idem exemple 1) 8 g
- Polyisobutène hydrogéné 28 g
- Polybutène 10 g
- Malate de diisostéaryle 5 g
- Octyldodécanol 5 g
- Lanoline oxypropylénée à 5 moles d 'oxyde de propylène 5 g
- Hectorite modifiée (Bentone) 0,8 g
- Homopolymère d'éthylène commercialisé sous la dénomination de "Polywax 500" par la Société BARECO 11 g
- Stéarate d'octacosanyle 4 g
- Huile de coco hydrogénée 5 g
- Polydiméthylsiloxane (5 cst) 9,54 g
- Pigments 8,66 g

Le rouge à lèvres obtenu présente une grande douceur à l'application et conduit à l'obtention sur les lèvres d'un film léger, confortable et non gras.

### EXEMPLE 4 : Fond de teint

On prépare un fond de teint sous forme d'une émulsion eau-dans-l'huile (E/H) à l'aide des ingrédients suivants :

### Phase grasse A

- Isostéarate de poly 4-glycéryle et cétyldiméthicone copolyol et laurate d'hexyle commercialisé sous la dénomination de "Abil WE09" par la Société GOLDSCHMIDT 5 g
- Alkylsilicone fluorée (idem Exemple 1) 15 g
- Pigments (oxyde de fer et dioxyde de titane) enrobés par des polydiméthylsiloxanes 7 g

### Phase aqueuse B

- Conservateur q.s.
- Eau q.s.p 100 g

Cette émulsion E/H est obtenue en dispersant les pigments dans la *phase grasse* à température ambiante. On y incorpore ensuite sous agitation, à l'aide d'une turbine de type Moritz, à la vitesse de 3.000 t/mn, la *Phase aqueuse B.*

### EXEMPLE 5 :

On prépare un vernis à ongles nacré à l'aide des ingrédients suivants :
- Nitrocellulose 12 g
- Résine alkyde 5 g
- Acétyl citrate de tributyle 3 g
- Alkylsilicone fluorée (idem Exemple 1) 3 g
- Alcool isopropylique 8 g
- Hectorite 1 g
- Pigments nacrants 0,5 g
- Acétate d'éthyle/Acétate de butyle q.s.p. 100 g

Ce vernis est obtenu sous forte agitation et sous pression en dispersant l'hectorite dans une partie de nitrocellulose et le mélange des acétates puis on ajoute le reste des ingrédients.

## Revendications

1. Composition cosmétique ou dermatologique **caractérisée par le fait qu'**elle contient en tant qu'ingrédient au moins une alkylsilicone fluorée répondant à l'une des formules (I) et/ou (II) suivantes : 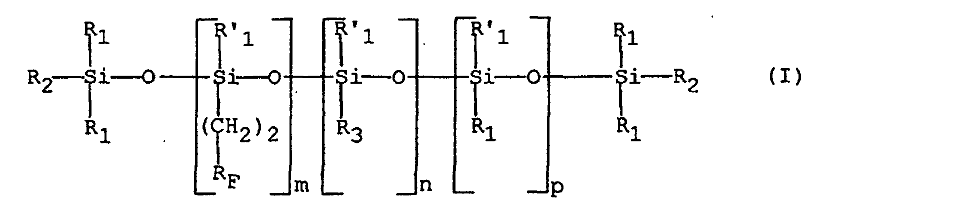 dans laquelle :
R₁ et R'₁ représentent indépendamment un radical alkyle, linéaire ou ramifié, ayant de 1 à 6 atomes de carbone, ou un radical phényle,
R₂ représente R₁, -OH, ou -(CH₂)_{f}-R_{F}, f étant un nombre entier allant de 0 à 10,
R₃ représente un radical alkyle, linéaire ou ramifié, ayant de 6 à 22 atomes de carbone,
R_{F} représente un radical de formule -(CF₂)_{q}-CF₃, q étant un nombre entier allant de 0 à 15,
m et n représentent un nombre entier allant de 1 à 50, et
p représente un nombre entier allant de 0 à 2000, 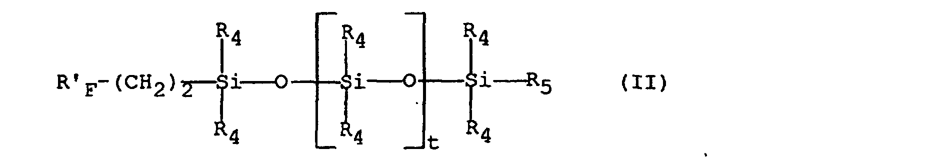
dans laquelle :
R₄ représente un radical alkyle, linéaire ou ramifié, ayant de 1 à 6 atomes de carbone, ou un radical phényle,
R₅ représente un radical alkyle, linéaire ou ramifié, ayant de 6 à 22 atomes de carbone,
R'_{F} représente un radical de formule -(CF₂)ₛ-CF₃, s étant un nombre entier allant de 0 à 15, et
t représente un nombre entier allant dé 1 à 2000.

2. Composition selon la revendication 1, **caractérisée par le fait que** ladite alkylsilicone fluorée répond à la formule (I) dans laquelle :
R₁, R'₁ et R₂ représentent le radical méthyle,
R₃ représente un radical alkyle linéaire ayant de 6 à 22 atomes de carbone,
m et n sont des nombres entiers allant de 1 à 20, et
q est un nombre entier allant de 1 à 13.

3. Composition selon la revendication 1, **caractérisée par le fait que** ladite alkylsilicone fluorée répond à la formule (II) dans laquelle :
R₄ représente le radical méthyle,
R₅ représente un radical alkyle linéaire ayant de 6 à 22 atomes de carbone, et
s représente un nombre entier allant de 1 à 13.

4. Composition selon l'une quelconque des revendications précédentes, **caractérisée par le fait que** ladite alkylsilicone fluorée est présente en une proportion allant de 0,1 à 99 % en poids par rapport au poids total de la composition.

5. Composition selon l'une quelconque des revendications précédentes, **caractérisée par le fait que** ladite composition est une composition anhydre contenant une phase grasse en une proportion allant de 0,1 à 99,9 % en poids par rapport au poids total de la composition, ladite phase grasse contenant :
- (i) de 0,1 à 99,9 % en poids par rapport au poids total de la composition, d'une alkylsilicone fluorée de formule (I) et/ou (II), et
- (ii) de 0 à 99,8 % en poids par rapport au poids total de la composition d'au moins un corps gras liquide, solide ou semi-solide.

6. Composition selon la revendication 5, **caractérisée par le fait que** ledit corps gras est choisi dans le groupe constitué par l'isododécane, le polyisobutène hydrogéné, le squalane, l'isononancate d'isononyle, les cyclotétra- et penta-diméthicones, la phényltriméthicone, les homopolymères d'éthylène, les copolymères d'éthylène et d'au moins un monomère répondant à la formule (III) suivante :
CH₂=CH-R₈ (III)
dans laquelle :
R₈ représente un radical alkyle ayant de 1 à 30 atomes de carbone, un radical aryle ou aralkyle,
lesdits homopolymères et copolymères ayant une masse moléculaire moyenne en poids comprise entre 200 et 1000,
les cires microcristallines, l'ozokérite, la cire d'abeilles, la cire de candelilla et le propionate d'arachidyle.

7. Composition selon l'une quelconque des revendications précédentes, **caractérisée par le fait qu'**elle se présente sous la forme d'un gel huileux, d'une poudre compactée, d'une poudre coulée ou d'un stick.

8. Composition selon la revendication 7, **caractérisée par le fait que** le stick est un rouge à lèvres contenant au moins un colorant et/ou pigment en une proportion allant de 0,1 à 15 % en poids par rapport au poids total de la composition sous forme de rouge à lèvres.

9. Composition selon l'une quelconque des revendications 1 à 4, **caractérisée par le fait qu'**elle se présente sous forme d'une dispersion stable du type eau-dans-l'huile ou huile-dans-l'eau, comprenant (i) une phase grasse en une proportion allant de 0,1 à 50 % en poids par rapport au poids total de la composition, ladite phase grasse contenant une alkylsilicone fluorée de formule (I) et/ou (II) selon l'une quelconque des revendications 1 à 3 en une proportion allant de 0,1 à 50 % en poids par rapport au poids total de la composition, (ii) une phase aqueuse en une proportion allant de 50 à 98,9 % en poids par rapport au poids total de la composition, et (iii) au moins un agent émulsionnant en une proportion allant de 1 à 10 % en poids par rapport au poids total de la composition.

10. Composition selon la revendication 9, **caractérisée par le fait que** ladite phase grasse contient au moins un corps gras tel que défini selon la revendication 6.

11. Composition selon la revendication 9, **caractérisée par le fait que** ledit agent émulsionnant est choisi dans le groupe constitué par les alkyl- ou alcoxydiméthicone copolyols de formule générale suivante : 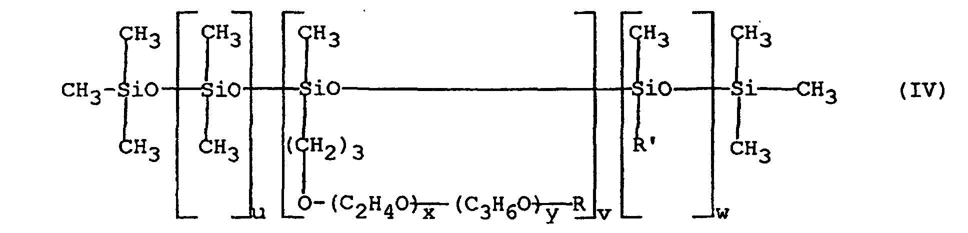 dans laquelle :
R est un atome d'hydrogène, un alkyle en C₁-C₁₆, un alcoxy ou acyle,
R' est un radical alkyle ou alcoxy en C₈-C₂₂,
u = 0 à 200,
v = 1 à 40,
w = 1 à 100,
le poids moléculaire du radical 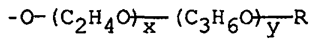 est de 250 à 2000, x et y étant choisis de telle sorte que le rapport en poids des groupes oxyéthylène/oxypropylène soit compris entre 100:0 et 20:80.

12. Composition selon l'une quelconque des revendications 1 à 4, **caractérisée par le fait qu'**elle est une composition pour le maquillage ou les soins des ongles, celle-ci contenant une alkylsilicone fluorée de formule (I) et/ou (II) en une proportion allant de 0,1 à 99,9 % en poids par rapport au poids total de la composition.

13. Composition selon la revendication 12, **caractérisée par le fait qu'**elle se présente sous forme d'un vernis à ongles et contient :
(i) une alkylsilicone fluorée de formule (I) et/ou (II) en une proportion allant de 2 à 40 % en poids par rapport au poids total du vernis,
(ii) un mélange solvant pour vernis, et
(iii) une substance filmogène.

14. Composition selon la revendication 13, **caractérisée par le fait que** le mélange solvant est présent en une proportion allant de 55 à 90 % en poids et la substance filmogène en une proportion allant de 5 à 35 % en poids par rapport au poids total de la composition sous forme de vernis à ongles.

15. Composition selon l'une quelconque des revendications 1 à 4, **caractérisée par le fait que** ladite composition est une composition capillaire comprenant, dans un véhicule cosmétique choisi parmi les solutions alcooliques et hydroalcooliques, au moins une alkylsilicone fluorée de formule (I) et/ou (II) en une proportion allant de 0,5 à 40 % en poids par rapport au poids total de la composition.

16. Composition selon l'une quelconque des revendications précédentes, **caractérisée par le fait que** ladite composition contient en outre au moins un adjuvant cosmétique conventionnel choisi dans le groupe constitué par les charges, les filtres solaires UVA et/ou UVB, les vitamines, les hormones, les agents antioxydants, les conservateurs, les colorants, les pigments, les parfums, les épaississants, les agents hydratants, les agents humectants, les polymères anioniques, non-ioniques ou amphotères et les substances actives cosmétiques ou dermatologiques.

## Patentansprüche

1. Kosmetische oder dermatologische Zubereitung, **dadurch gekennzeichnet, dass** als Bestandteil mindestens ein fluoriertes Alkylsilikon enthält, entsprechend einer der folgenden Formeln (I) und/oder (II): 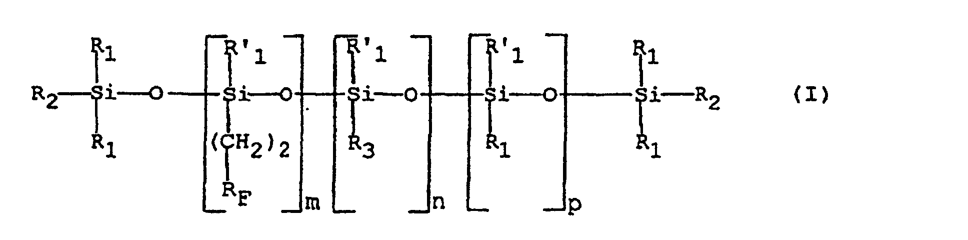 worin:
R₁ und R'₁ unabhängig einen linearen oder verzweigten Alkylrest mit 1 bis 6 Kohlenstoffatomen oder einen Phenylrest darstellen,
R₂ für R₁, -OH oder -(CH₂)_{f}-R_{F} steht, worin f eine ganze Zahl von 0 bis 10 bedeutet,
R₃ einen linearen oder verzweigten Alkylrest mit 6 bis 22 Kohlenstoffatomen darstellt,
R_{F} einen Rest der Formel -(CF₂)_{q}-CF₃ darstellt, worin q eine ganze Zahl von 0 bis 15 bedeutet,
m und n eine ganze Zahl von 1 bis 50 darstellen, und
p eine ganze Zahl von 0 bis 2000 bedeutet, 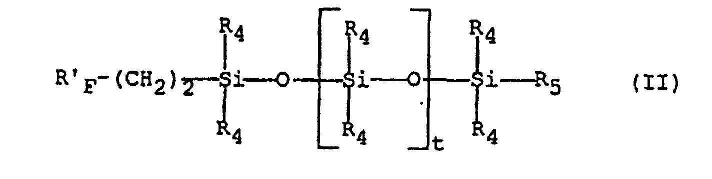
worin:
R₄ einen linearen oder verzweigten Alkylrest mit 1 bis 6 Kohlenstoffatomen oder einen Phenylrest darstellt,
R₅ einen linearen oder verzweigten Alkylrest mit 6 bis 22 Kohlenstoffatomen darstellt,
R'_{F} einen Rest der Formel -(CF₂)ₛ-CF₃ darstellt, worin s eine ganze Zahl von 0 bis 15 ist, und
t eine ganze Zahl von 1 bis 2000 bedeutet.

2. Zubereitung gemäß Anspruch 1, **dadurch gekennzeichnet, dass** das fluorierte Alkylsilikon der Formel (I) entspricht, worin:
R₁, R'₁ und R₂ einen Methylrest darstellen,
R₃ einen linearen Alkylrest mit 6 bis 22 Kohlenstoffatomen darstellt,
m und n ganze Zahlen von 1 bis 20 sind, und
q eine ganze Zahl von 1 bis 13 ist.

3. Zubereitung gemäß Anspruch 1, **dadurch gekennzeichnet, dass** das fluorierte Alkylsilikon der Formel (II) entspricht, worin:
R₄ den Methylrest darstellt,
R₅ einen linearen Alkylrest mit 6 bis 22 Kohlenstoffatomen darstellt, und
s eine ganze Zahl von 1 bis 13 ist.

4. Zubereitung gemäß einem der vorstehenden Ansprüche, **dadurch gekennzeichnet, dass** das fluorierte Alkylsilikon in einer Menge von 0,1 bis 99 Gew.-% vorliegt, bezogen auf das Gesamtgewicht der Zubereitung.

5. Zubereitung gemäß einem der vorstehenden Ansprüche, **dadurch gekennzeichnet, dass** die Zubereitung eine wasserfreie Zubereitung ist, enthaltend eine Fettphase in einer Menge von 0,1 bis 99,9 Gew.-%, bezogen auf das Gesamtgewicht der Zubereitung, wobei die Fettphase enthält:
- (i) von 0,1 bis 99,9 Gew.-%, bezogen auf das Gesamtgewicht der Zubereitung, eines Alkylsilikons der Formel (I) und/oder (II), und
- (ii) 0 bis 99,8 Gew.-%, bezogen auf das Gesamtgewicht der Zubereitung, mindestens eines flüssigen, festen oder halbfesten Fettkörpers.

6. Zubereitung gemäß Anspruch 5, **dadurch gekennzeichnet, dass** der Fettkörper ausgewählt ist aus der Gruppe, bestehend aus Isodecan, hydriertem Polyisobuten, Squalen, Isononylisononanoat, Cyclotetra- und -pentadimethiconen, Phenyltrimethicon, Homopolymeren von Ethylen, Copolymeren von Ethylen und mindestens einem Monomeren, entsprechend der folgenden Formel (III):
CH₂=CH-R₈ (III)
worin:
R₈ einen Alkylrest mit 1 bis 30 Kohlenstoffatomen, einen Arylrest oder Aralkylrest darstellt,
wobei die Homopolymere und Copolymere ein mittleres Molekulargewicht zwischen 200 und 1000 aufweisen,
mikrokristallinen Wachsen, Ozokerit, Bienenwachs, Candelillawachs und Arachidylpropionat.

7. Zubereitung gemäß einem der vorstehenden Ansprüche, **dadurch gekennzeichnet, dass** sie in Form eines öligen Gels, eines Kompaktpuders, eines gegossenen Puders oder eines Stiftes (Sticks) vorliegt.

8. Zubereitung gemäß Anspruch 7, **dadurch gekennzeichnet, dass** der Stift ein Lippenstift ist, enthaltend mindestens ein farbgebendes Mittel und/oder Pigment in einer Menge von 0,1 bis 15 Gew.-%, bezogen auf das Gesamtgewicht der Zubereitung in Form des Lippenstiftes.

9. Zubereitung gemäß einem der Ansprüche 1 bis 4, **dadurch gekennzeichnet, dass** sie in Form einer stabilen Dispersion vom Typ Wasser-in-Öl oder Öl-in-Wasser vorliegt, enthaltend (i) eine Fettphase in einer Menge von 0,1 bis 50 Gew.-%, bezogen auf das Gesamtgewicht der Zubereitung, wobei die Fettphase ein fluoriertes Alkylsilikon der Formel (I) und/oder (II) gemäß einem der Ansprüche 1 bis 3 in einer Menge von 0,1 bis 50 Gew.-%, bezogen auf das Gesamtgewicht der Zubereitung, enthält, (ii) eine wässrige Phase in einer Menge von 50 bis 98,9 Gew.-%, bezogen auf das Gesamtgewicht der Zubereitung, und (iii) mindestens einen Emulgator in einer Menge von 1 bis 10 Gew.-%, bezogen auf das Gesamtgewicht der Zubereitung.

10. Zubereitung gemäß Anspruch 9, **dadurch gekennzeichnet, dass** die Fettphase mindestens einen Fettkörper enthält, wie er gemäß Anspruch 6 definiert ist.

11. Zubereitung gemäß Anspruch 9, **dadurch gekennzeichnet, dass** das Emulgiermittel ausgewählt ist aus der Gruppe, bestehend aus den Alkyl- oder Alkoxydimethicon-Copolyolen der folgenden allgemeinen Formel: 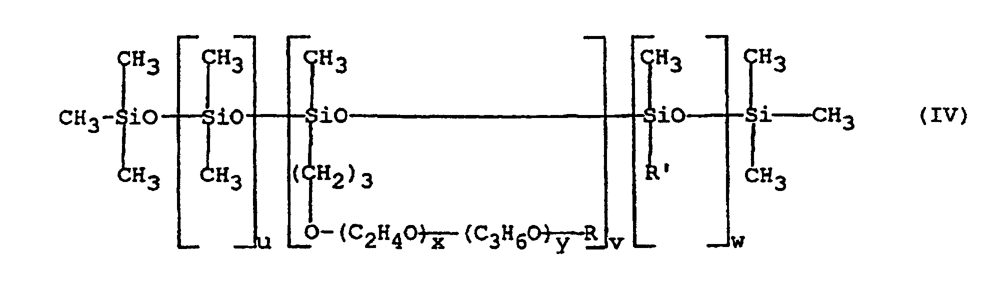 worin:
R ein Wasserstoffatom, ein C₁-C₁₆-Alkyl, ein Alkoxy oder Acyl ist,
R' ein Alkylrest oder Alkoxyrest mit C₈-C₂₂ ist,
u = 0 bis 200
v = 1 bis 40,
w = 1 bis 100,
wobei das Molekulargewicht des Restes 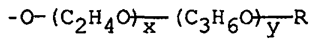 von 250 bis 2000 beträgt, x und y so gewählt sind, dass das Gewichtsverhältnis der Ethylenoxid/Propylenoxid-Gruppen zwischen 100:0 und 20:80 liegt.

12. Zubereitung gemäß einem der Ansprüche 1 bis 4, **dadurch gekennzeichnet, dass** es sich um eine Zubereitung für das Schminken oder die Pflege der Nägel handelt, die ein fluoriertes Alkylsilikon der Formel (I) und/oder (II) in einer Menge von 0,1 bis 99,9 Gew.-% enthält, bezogen auf das Gesamtgewicht der Zubereitung.

13. Zubereitung gemäß Anspruch 12, **dadurch gekennzeichnet, dass** sie in Form eines Nagellackes vorliegt und enthält:
(i) ein fluoriertes Alkylsilikon der Formel (I) und/oder (II) in einer Menge von 2 bis 40 Gew.-%, bezogen auf das Gesamtgewicht des Lackes,
(ii) ein Lösungsmittelgemisch für Lacke, und
(iii) eine filmbildende Substanz.

14. Zubereitung gemäß Anspruch 13, **dadurch gekennzeichnet, dass** die Lösungsmittelmischung in einer Menge von 55 bis 90 Gew.-% vorhanden ist und dass die filmbildende Substanz in einer Menge von 5 bis 35 Gew.-% vorhanden ist, bezogen auf das Gesamtgewicht der Zubereitung in Form eines Nagellackes.

15. Zubereitung gemäß einem der Ansprüche 1 bis 4, **dadurch gekennzeichnet, dass** die Zubereitung eine Zubereitung für die Haare ist, enthaltend in einem kosmetisch annehmbaren Träger, ausgewählt unter alkoholischen und hydroalkoholischen Lösungen, mindestens ein fluoriertes Alkylsilikon der Formel (I) und/oder (II) in einer Menge von 0,5 bis 40 Gew.-%, bezogen auf das Gesamtgewicht der Zubereitung.

16. Zubereitung gemäß einem der vorstehenden Ansprüche, **dadurch gekennzeichnet, dass** die Zubereitung zusätzlich enthält mindestens ein übliches kosmetisches Adjuvans oder Hilfsmittel, ausgewählt aus der Gruppe, bestehend aus Zuschlägen, UVA- und/oder UVB-Sonnenfiltern, den Vitaminen, den Hormonen, den Antioxidantien, den Konservierungsmitteln, den farbgebenden Mitteln, den Pigmenten, den Parfümen, den Verdickungsmitteln, den Hydrierungsmitteln, den Feuchthaltemitteln, den anionischen, nichtionischen oder amphoteren Polymeren und kosmetisch oder dermatologisch aktiven Substanzen.

## Claims

1. Cosmetic or dermatological composition, **characterized in that** it contains, as ingredient, at least one fluoroalkylsilicone corresponding to one of the formulae (I) and/or (II) below: 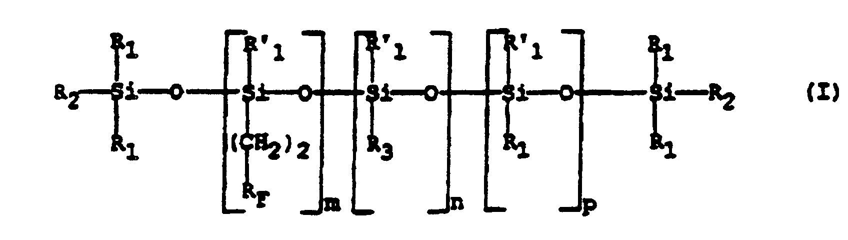 in which:
R₁ and R'₁ independently represent a linear or branched alkyl radical having from 1 to 6 carbon atoms or a phenyl radical,
R₂ represents R₁, -OH or -(CH₂)_{f}-R_{F}, f being an integer ranging from 0 to 10,
R₃ represents a linear or branched alkyl radical having from 6 to 22 carbon atoms,
R_{F} represents a radical of formula -(CF₂)_{q}-CF₃, q being an integer ranging from 0 to 15,
m and n represent an integer ranging from 1 to 50, and
p represents an integer ranging from 0 to 2000, 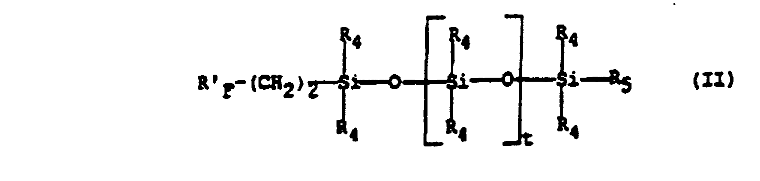
in which:
R₄ represents a linear or branched alkyl radical having from 1 to 6 carbon atoms, or a phenyl radical,
R₅ represents a linear or branched alkyl radical having from 6 to 22 carbon atoms,
R'_{F} represents a radical of formula -(CF₂)ₛ-CF₃, s being an integer ranging from 0 to 15, and
t represents an integer ranging from 1 to 2000.

2. Composition according to Claim 1, **characterized in that** the said fluoroalkylsilicone corresponds to formula (I) in which:
R₁, R'₁ and R₂ represent a methyl radical,
R₃ represents a linear alkyl radical having from 6 to 22 carbon atoms,
m and n are integers ranging from 1 to 20, and
q is an integer ranging from 1 to 13.

3. Composition according to Claim 1, **characterized in that** the said fluoroalkylsilicone corresponds to formula (II) in which:
R₄ represents a methyl radical,
R₅ represents a linear alkyl radical having from 6 to 22 carbon atoms, and
s represents an integer ranging from 1 to 13.

4. Composition according to any one of the preceding claims, **characterized in that** the said fluoroalkylsilicone is in a proportion ranging from 0.1 to 99% by weight relative to the total weight of the composition.

5. Composition according to any one of the preceding claims, **characterized in that** the said composition is an anhydrous composition containing a fatty phase in a proportion ranging from 0.1 to 99.9% by weight relative to the total weight of the composition, the said fatty phase containing:
- (i) from 0.1 to 99.9% by weight, relative to the total weight of the composition, of a fluoroalkylsilicone of formula (I) and/or (II), and
- (ii) from 0 to 99.8% by weight, relative to the total weight of the composition, of at least one liquid, solid or semisolid fatty substance.

6. Composition according to Claim 5, **characterized in that** the said fatty substance is chosen from the group consisting of isododecane, hydrogenated polyisobutene, squalane, isononyl isononanoate, cyclotetra- and -penta-dimethicones, phenyltrimethicone, ethylene homopolymers, copolymers of ethylene and of at least one monomer corresponding to formula (III) below:
CH₂=CH-R₈ (III)
in which:
R₈ represents an alkyl radical having from 1 to 30 carbon atoms or an aryl or aralkyl radical,
the said homopolymers and copolymers having a weight-average molecular mass of between 200 and 1000,
microcrystalline waxes, ozokerite, beeswax, candelilla wax and arachidyl propionate.

7. Composition according to any one of the preceding claims, **characterized in that** it is in the form of an oily gel, a compacted powder, a cast powder or a stick.

8. Composition according to Claim 7, **characterized in that** the stick is a lipstick containing at least one dye and/or pigment in a proportion ranging from 0.1 to 15% by weight relative to the total weight of the composition in lipstick form.

9. Composition according to any one of Claims 1 to 4, **characterized in that** it is in the form of a stable dispersion of the water-in-oil or oil-in-water type, comprising (i) a fatty phase in a proportion ranging from 0.1 to 50% by weight relative to the total weight of the composition, the said fatty phase containing a fluoroalkylsilicone of formula (I) and/or (II) according to any one of Claims 1 to 3 in a proportion ranging from 0.1 to 50% by weight relative to the total weight of the composition, (ii) an aqueous phase in a proportion ranging from 50 to 98.9% by weight relative to the total weight of the composition, and (iii) at least one emulsifier in a proportion ranging from 1 to 10% by weight relative to the total weight of the composition.

10. Composition according to Claim 9, **characterized in that** the said fatty phase contains at least one fatty substance as defined according to Claim 6.

11. Composition according to Claim 9, **characterized in that** the said emulsifier is chosen from the group consisting of alkyl- or alkoxy-dimethicone copolyols of the following general formula: 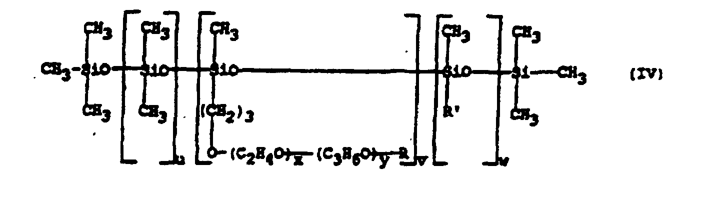 in which:
R is a hydrogen atom, a C₁-C₁₆ alkyl, an alkoxy or an acyl,
R' is a C₈-C₂₂ alkyl.or alkoxy radical,
u = 0 to 200,
v = 1 to 40,
w = 1 to 100,
the molecular weight of the radical -O-(C₂H₄O)ₓ-(C₃H₆O)_{y}-R is from 250 to 2000, x and y being chosen such that the weight ratio of the oxyethylene/oxypropylene groups is between 100:0 and 20:80.

12. Composition according to any one of Claims 1 to 4, **characterized in that** it is a make-up or nailcare composition containing a fluoroalkylsilicone of formula (I) and/or (II) in a proportion ranging from 0.1 to 99.9% by weight relative to the total weight of the composition.

13. Composition according to Claim 12, **characterized in that** it is in the form of a nail varnish and contains:
(i)a fluoroalkylsilicone of formula (I) and/or (II) in a proportion ranging from 2 to 40% by weight relative to the total weight of the varnish,
(ii)a solvent mixture for varnishes, and
(iii)a film-forming substance.

14. Composition according to Claim 13, **characterized in that** the solvent mixture is present in a proportion ranging from 55 to 90% by weight and the film-forming substance is present in a proportion ranging from 5 to 35% by weight relative to the total weight of the composition in nail varnish form.

15. Composition according to any one of Claims 1 to 4, **characterized in that** the said composition is a hair composition comprising, in a cosmetic vehicle chosen from alcholic and aqueous-alcoholic solutions, at least one fluoroalkylsilicone of formula (I) and/or (II) in a proportion ranging from 0.5 to 40% by weight relative to the total weight of the composition.

16. Composition according to any one of the preceding claims, **characterized in that** the said composition also contains at least one conventional cosmetic adjuvant chosen from the group consisting of fillers, UVA and/or UVB sunscreens, vitamins, hormones, antioxidants, preserving agents, dyes, pigments, fragrances, thickeners, moisturizers, wetting agents, anionic, nonionic or amphoteric polymers and cosmetic or dermatological active substances.
